Europäisches Patentamt

**European Patent Office**

Office européen des brevets

(11) Publication number: **0 214 281 B1**

# EUROPEAN PATENT SPECIFICATION

(45) Date of publication of patent specification: **09.09.92**    (51) Int. Cl.5: **A61K  37/02**

(21) Application number: **86902132.9**

(22) Date of filing: **06.03.86**

(86) International application number:
**PCT/US86/00475**

(87) International publication number:
**WO 86/05096 (12.09.86 86/20)**

(54) **PROTEIN ABSORPTION ENHANCING AGENTS.**

(30) Priority: **06.03.85 US 708845**
**27.03.85 US 716705**
**01.10.85 US 782441**

(43) Date of publication of application:
**18.03.87 Bulletin  87/12**

(45) Publication of the grant of the patent:
**09.09.92 Bulletin  92/37**

(84) Designated Contracting States:
**AT BE CH DE FR GB IT LI LU NL SE**

(56) References cited:

**N, Chemical Abstracts Vol. 97 Published
1982 Abstract 195,021R**

**N, Chemical Abstracts Vol. 96 Published
1982 Abstract 83,378K**

**N, Chemical Abstracts Vol. 99 Published
1983 Abstract 20521t**

(73) Proprietor: **SURVIVAL TECHNOLOGY, INC.**
**8101 Glenbrook Road**
**Bethesda, MD 20814(US)**

(72) Inventor: **SARNOFF, Stanley, Jay**
**7507 Hampden Lane**
**Bethesda, MD 20814(US)**
Inventor: **SOBEL, Burton, E.**
**444 Baker Avenue**
**Webster Grove, MO 26319(US)**

(74) Representative: **UEXKÜLL & STOLBERG Patentanwälte**
**Beselerstrasse 4**
**W-2000 Hamburg 52(DE)**

## Description

This application relates to a package containing a protein having therapeutic properties and an agent capable of enhancing the absorption of the protein.

According to the present invention a package has been developed for enhancement of absorption of proteins with medicinal properties administered by intramuscular injection to rapidly achieve high concentrations of the injected protein in the circulating plasma permitting rapid implementation of therapeutic effects without the need for intravenous injection or infusion. In an exemplary embodiment there is provided a vehicle in which the protein is administered in a vehicle comprising an absorption-enhancement compound that markedly increases permeation of proteins tested into the vascular space leading to high blood levels within two minutes after injection in tests as set forth below.

The invention is set forth in the context of treatment of coronary prone individuals in the throes of a suspected myocardial infarction in such a way as to minimize damage to the heart muscle and, more particularly, to improvements in such treatments enabling the same to be commenced at the earliest possible time, even before direct qualified personal care of the individual can be established.

When a clot forms in a blood vessel, the body organ being supplied with blood by that blood vessel is compromised or totally deprived of blood supply. Depending on the blood vessel in which this occurs, the threat to the life of the individual is either small or very great as in the circumstances to be addressed by the material below, i.e. certain life threatening circumstances. Clot formation in a vessel is described as thrombosis. Substances which dissolve thrombi are called thrombolytic substances. When a coronary artery clot is dissolved, the resultant establishment of blood flow to the heart is called reperfusion.

Examples of life threatening or very serious clot formation in arterial vessels are cerebral thrombosis, renal thrombosis, opthalmic artery thrombosis, and very importantly, thrombosis of a coronary artery. In approximately 85% to 90% of cases of acute myocardial infarction (coronary heart attack), a thrombus is found in the coronary artery preventing blood from flowing to the heart muscle (myocardium) and supplying it with essential oxygen and other nutrients. A consequence of a thrombus or clot forming in a coronary artery is the danger to the myocardium (heart muscle tissue that does the pumping of blood). Heart muscle deprived of it's blood supply does not die immediately but does promptly begin the process of becoming dead. The extent of the damage which is done to the heart muscle is, therefore, a function of the time during which the supply of blood to the infarct zone is restricted by the occuluding thrombus

Heretofore, the procedures undertaken to actually establish reperfusion to the infarct zone have generally been undertaken in a hospital environment or equivalent. The so-called "prehospital" treatment was, in general, directed toward keeping the patient alive and getting the patient into the hospital environment as soon as possible so that treatment minimizing the heart muscle damage could be accomplished.

The treatment undertaken in the hospital environment involves certain procedures for establishing reperfusion in the infarct zone of the patient's heart. When immediate surgery was not clearly indicated, the establishment of reperfusion was accomplished by procedures which had the effect of unblocking the occlusion. The available procedures included mechanical catheterization and the administration of thrombolytic agents. Known thrombolytic agents, such as streptokinase or urokinase required intracoronary infusion or the slow infeed of the agent within the vessel at the site of occlusion by means of a catheter. In recent years, intravenous infusion of streptokinase has been shown to be effective.

More recently a substance called tissue-type plasminogen activator or t-PA has been utilized experimentally. (The New England Journal of Medicine, March 8, 1984, Volume 310, No. 10, pages 609-613). Unlike other plasminogen activators, such as streptokinase or urokinase, t-PA--which is found in only small amounts in the body--acts specifically on clots and not on other relevant proteins in the blood, when maintained at appropriate and effective levels.

A 1984 Commentary found in Biochemical Pharmacology, Volume 33, No. 12, pages 1831-1838 entitled "Coronary Thrombolysis: Pharmacological Considerations With Emphasis On Tissue-Type Plasminogen Activator (t-PA)" contains the following conclusionary statement:

"Selection of pharmacological agents for induction of coronary thrombolysis has been determined largely by availability. Unfortunately, both streptokinase and urokinase induce a systemic lytic state with depletion of circulating fibrinogen, plasminogen, and $\alpha$ 2-antiplasmin, and accumulation of fibrin degradation products. All of these factors conspire to set the stage for hemorrhage with a risk of serious bleeding. Intravenous administration of these agents is limited by a lower success rate, in part because the upper bound of dose is constrained by the risk of inducing a severe systemic lytic state.

The probability that progress in recombinant DNA technology will lead to widespread availability of tissue-type plasminogen activiator is particularly exciting because of the clot specific properties of t-PA. For coronary thrombolysis its potential advantages include: safety and efficacy of intravenous administration of high doses; effective clot lysis without induction of a systemic lytic state; prompt implementation without the need for extensive characterization of the coagulation and fibrinolytic systems in each patient prior to and during therapy; avoidance of frank allergic reactions or variations in dose-response relation due to immune complex formation; ease of minute-by-minute adjustment of dosage and prompt termination of fibrinolysis when needed because of the short biological half-like of t-PA and the lack of induction of a systemic lytic state."

The promise attributable to t-PA administration was discussed at a news conference at a meeting of the Americal Heart Association and reported by the New York Times on November 16, 1983, in an article entitiled, "Protein Of Cancer Cells Used To Halt Coronaries." The article refers to injection of t-PA by stating the following: "The protein t-PA can simply be injected into the vein in the art of the patient seized by a myocardial infarction or heart attack, and it travels through the blood to dissolve a clot, in much the same way as Draino clears up stopped plumbing."

The article further indicated under the subheading "Hopes For Future Application" that many physicians have expressed excitement about research into the use of t-PA to treat heart attacks because they hope that some day it may be used in emergency rooms and ambulances to stop heart attacks at their earliest stages before they kill or cause permanent damage. Under the "Hopes For Future Application" subheading there is also included the following paragraph: "Dr. Burton E. Sobel Of Washington University, one of the researchers, speculated that patients might some day carry a vial with them so that the drug could be injected immediately after they felt chest pain and other early symptoms of a heart attack."

In medical parlance, a vial is a container for a quantity of liquid medicine or diluent having a rubber stopper capable of being pierced by a hypodermic needle of a syringe to enable the operator of the syringe to withdraw a predetermined dosage of the liquid from the vial. In the case of t-PA as currently used, the dosage could then be injected into the mother liquid container of an infusion assembly. The necessity to administer the drug by intravenous infusion or by intravenous injection presents a significant barrier to self-administration from a practical view point, particularly when considering the disconcerting circumstances of the individual undergoing the symptoms of a myocardial infarction.

The development of a package for use in an effective self-administration procedure for t-PA sufficient to enable its utilization by a targeted coronary prone individual immediately following onset of symptoms, would materially increase the potential efficacy of t-PA as a thromobolytic agent by insuring its use at the earliest possible time often before irreversible heart muscle damage has occurred, and, at the same time, enable for a treatment of the pre-hospital or pre-ambulance type which for the first time is directly effective to minimize heart muscle damage accompanying myocaridal infarction. It is an object of the present invention to provide such a package.

A number of proteins with life-saving, therapeutic properties presently must be administered exclusively intravascularly to achieve therapeutic blood levels. Examples include activators of the clot-dissolving, fibrinolytic system including streptokinase and urokinase used for interrupting heart attacks in progress, and components of the coagulation system such as antihemophiliac globulin (factor VIII) to arrest bleeding in hemophiliacs. In many circumstances other proteins such as insulin used to treat diabetic coma must be administered intravenously to yield concentrations high enough to manifest the desired therapeutic effect of promptly enough to do so within the time-constraints of the medical emergency. Recently, as pointed out above, dissolution of clots in the coronary arteries giving rise to heart attacks has been achieved in experimental animals and patients by intravenous administration of tissue-type plasminogen activator (t-PA), a protein capable of activating the fibronolytic system at the clot surface without predisposing the patient to bleeding. Because the half-life in the circulation of t-PA is so short, continuous intravenous infusion has been required to avoid rapid disappearance of the protein from the circulation with consequently sub-therapeutic concentrations. In addition, intravenous administration has been deemed necessary because of the urgent need for rapid clot lysis to interrupt the heart attack in progress before death of a substantial amount of heart muscle.

In view of the foregoing a package has been developed containing an agent capable of enhancing absorption in the blood of protein, e.g. t-PA, and other large proteins injected intramuscularly. The package utilizes solubilization of the protein in a vehicle enriched with an agent enhancing absorption of the protein in the blood that elicits prompt permeation of the protein into the circulating blood pool. An additional objective of the package developed is retention of the pharmacologic and therapeutic properties of the protein so that the desired therapeutic effect can be elicited by intramuscular injection with enhanced absorption. A further objective is facilitation of absorption over 30 to 60 minutes so that therapeutic blood levels can be sustained under emergency conditions. Permeation of the proteins tested into the vascular spaces led to high blood levels within 2 minutes of injection. The vehicle developed facilitates absorption of all four proteins in the tests mentioned below and exerts no deleterious effects on the functional properties of the two proteins tested with clot lysing properties. The invention is not limited to the proteins employed in the specific examples but is believed applicable to other proteins as well, such as those mentioned above, e.g. insulin.

The package according to the invention contains (1) a protein having therapeutic properties and (2) an agent capable of enhancing the absorption of the protein in the blood in an amount effective to enhance the absorption of the protein when injected intramuscularly, wherein the absorption enhancing agent is a $C_{1-4}$ alkylamine, a di $C_{1-3}$ alkylamine, phenylethylamine, epinephrine, tyramine, mono-, di-, or tri-ethanolamine, propanolamine, methoxyamine, ethylenediamine, diethylenetriamine, or non-toxic salts thereof. Particularly, the absorption enhancing agent is (1) a $C_{1-4}$ alkylamine, (2) a di $C_{1-3}$ alkylamine, (3) a non-toxic salt of $C_{1-4}$ alkylamine or (4) a non-toxic salt of a di $C_{1-3}$ alkylamine.

Preferred absorption enhancing agents are (a) methylamine, (b) dimethylamine, (c) a non-toxic salt of methylamine, or (d) a non-toxic salt of dimethylamine. More preferred is methylamine or a non-toxic salt of dimethylamine. The most preferred absorption enhancing agent is methylaminehydrochloride. It is preferred that the protein having therapeutic properties is t-PA.

The components of the package according to the invention can be administered for example with a known emergency type automatic injector and the process comprises injecting the two medicament agents into the muscle tissue, e.g. after having received a decision to do so over the telephone from a qualified source and at a time prior to the establishment of direct contact qualified personal care.

t-PA is a large protein. It would not be expected that it would be absorbed into the blood stream in discernible quantities. Extra-vascular levels of protein are about 1/10 that of intravascular protein. It is thought that this is so because the capillary pores through which transport of protein can occur are small relative to the molecular size of protein and limit protein transport because of electrical charge. It was thus highly problematical as to whether a large protein such as t-PA, when given intramuscularly, i.e. outside the blood vessels, would find its way rapidly into the blood stream in discernible quantities. Application tests have indeed shown that by itself t-PA does not find its way rapidly into the blood stream in therapeutically significant quantities after intramuscular injection.

The actual treatment of the system must therefore include intramuscular injection of an absorption enhancing agent simultaneously or substantially simultaneously with the intramuscular injection of the protein, e.g. t-PA.

Augmentation of absorption of low molecular weight substances administered topically, subcutaneously, or intramuscularly has been achieved with vehicles such as dimethylsulfoxide (DMSO) and by enhancement of skeletal muscle blood and lymph flow.

However, DMSO has proven ineffective as an absorption enhancing agent for t-PA and other protein molecules.

In accordance with the principles of the present invention, the absorption rate of t-PA and other proteins in the blood is enhanced by utilizing with the t-PA or other protein dosage, a dosage of an absorption enhancing agent for t-PA or other protein. Preferably, the absorption enhancing agent is mixed in with the t-PA or other protein dosage to form a single mixed dosage which is then injected intramuscularly (i.m.). It is within the contemplation of the present invention to inject the absorption enhancing agent as a separate dosage within the same site as the separate dosage of t-PA or other protein, (e.g. U.S. patent 4,394,863).

The absorption enhancing agents used according to the invention are alkyl amines and dialkyl amines such as lower alkyl amines and dialkylamines, e.g. methylamine, dimethylamine, ethylamine, diethylamine, isopropylamine, sec.butylamine, diisopropylamine, propylamine, n-butylamine, aralkylamines, e.g. phenylethylamine, hydroxyaralkylamines, e.g. epinephrine and tyramine, hydroxyalkylamines, e.g. ethanolamine, diethanolamine, triethanolamine, propanolamine, and other amines such as methoxyamine. polyalkylene amines, e.g. ethylene diamine, diethylene triamine. These amines also can be used in the form of salts of non-toxic acids such as salts of the acids mentioned earlier, e.g. as the hydrochlorides. Also there can be used glucoseoxime.

Methylamine and dimethylamine also have the advantage that they do not induce methemoglobinemia. This was shown by injecting rabbits with (a) 0.63 molar methylamine hydrochloride and (b) 0.63 molar methylamine hydrochloride together with t-PA in an amount of 1 mg/kg body weight. In each case 1 ml of injectate was employed and the solutions had a pH of 6.5. When using methylamine (as the hydrochloride) t-PA levels were in the range of 220 to 230 ng/ml. With dimethylamine the levels of t-PA were somewhat lower but still elevated compared to the case when t-PA was administered without an absorption enhancing agent. Methoxyamine (as the hydrochloride) under the same conditions also enhanced the level of t-PA but produced methemoglobenemia. The time course of elevation of t-PA over 30 minutes with methylamine hydrochloride was virtually the same as with hyroxylamine hydrochloride. The levels were more than 10 times the plasma t-PA levels in the absence of the absorption enhancing agent.

Also while the simultaneous administration of t-PA or other proteins and absorption enhancing agents is primarily intended for human use, it is within the scope of the invention that they be administered to other mammals, e.g. dogs, cats, cattle, and horses. In accordance with the earlier teachings electrical stimulation of the muscle at the injection site can be employed in concert with the inclusion of an absorption-enhancing agent in the injectate in a number of the following examples using intramuscular injection. Electrical stimulation augments and enhances the absorption of the absorption enhancing agent of the invention.

Although as pointed out in the earliest parent application an automatic injector device suitable for intramuscular self-administration of t-PA or other protein can be employed. EP: provided by EP 191814. The materials and methods described in examples are also relevant with regard to the use of the protein absorption enhancing agents according to the present invention.

To date, t-PA and other activators of the fibrinolytic system have been given only by direct injection into the blood stream. The package according to the invention enables for the administration of t-PA with an alternative means potentially amendabale to prompt implementation by paramedical personnel or by telephonically supervised patients at high risk previously instructed in self-medication procedures.

Methylamine was employed as the hydrochloride to enhance absorption of t-PA in rabbits. Absorption was measured after intramuscular injections of 2 to 3 mg of concentrated t-PA (50 mg/ml). Methylamine hydrochloride (0.63 molar) plus electrical field stimulation elected blood levels of rt-PA within 5 minutes after intramuscular injection of rt-PA, with functional and immunologic activity similar to that achieved with hydroxylamine (as the hydrochloride), (specifically 129 vs. 137 mg/ml/mg rt-PA). With the use of methylamine hydrochloride there was neither methemoglobinemia nor hemodynamic derangements occurred, oxygen saturation remained unchanged and only modest local inflammation and interstitial hemorrhage were evident microscopically in the injection site after 48 hours. Vasodilators, hypertonic media, reduced amounts of hydroxylamine hydrochloride or rt-PA alone led to much lower blood levels of rt-PA (14, 65, 46 or 4 ng/me/mg rt-PA respectively).

Blood levels of t-PA comparable to those obtained in the present investigation induce coronary thrombolysis in experimental animals and patients without inducing a systemic lytic state predisposing to bleeding. The time course of elevation of plasma t-PA after facilitated intramuscular absorption is particuarly favorable because of its sharp peak. With the envisioned application of an appropriate regimen, subjects would be under direct medical care soon after self-medication with an automatic injector or treatment by relatives of paramedical personnel. Thus, as the blood levels declined promptly after intramuscularly administered t-PA had been given, intravenous infusions could be initiated along with anticoagulants or other measures taken to prevent reocclusion while definitive diagnostic information was being obtained.

The possibility that myocardial reperfusion induced by facilitated absorption of intramuscularly administered t-PA might give rise to reperfusion arrhythmias is easily managed in the setting of the cardiac catheterization laboratory or coronary care unit but can be potentially dangerous in the medically unattended patient. Thus, there is advantage in the concomitant administration of an antifibrillatory or anti-arrhythmic agent such as lidocaine or an alpha-adrenergic blocking agent as set forth in the parent application.

It has also been found that to prevent reocclusions or platelet aggregation it is desirable to either:

1. inhibit synthesis of thromboxane A *thromboxane $A_2$) with a thromboxane synthetase inhibitor, e.g. an imidazole such as 4-(2-[1H-imidazol-1-yl]ethoxy)-benzoic acid hydrochloride (dazoxiben)

2. introduce an antagonist for the receptor of the thromboxane A (thromboxane $A_2$) such as [1$\alpha$, 2$\beta$ (5Z), 3$\beta$ (1E), 4$\alpha$]-7-[3-(3-cyclohexyl-3-hydroxy-1-propenyl)-7-oxabicyclo[2.2.1]hept-2-yl]-5-heptenoic acid)(SQ 27,427)

3. introduce another inhibitor of platelet aggregation, e.g. aspirin®, indomethacin, naproxin, and sulfinpyrazone.

The agent for the prevention of reocclusions or platelet aggregations could be administered simultaneously or sequentially in either order with reference to the t-PA and absorption enhancing agent.

The agent for the prevention of reocclusions or platelet aggregations can be administered in conventional manner, e.g. intramuscularly, intravenously, or even orally.

The receptor antagonist or other agent for prevention of platelet reocclusions can be administered for example in an amount of 0.1-10 mg/kg body weight.

As stated previously the invention is also useful to enhance the absorption of other proteins with medicinal properties by administering intramuscularly. Such proteins include for example bovine serum albumin, human serum albumin and insulin. This is shown by the following experiments:

Two proteins, bovine serum albumin (BSA) and human serum albumin (HSA), were labeled with iodine-125 ($^{125}$I) so that absorption into the circulation could be characterized by assay of radioactivity in trichloroacetic acid precipitates of plasma.

BSA and HSA proteins were labeled radioactively with iodine-125 ($^{125}$I) by the Bolton and Hunter method to form a radioligand on tyrosine constituents. In order to verify the independence of the results on the nature of the radioligand used, a second procedure was employed in which a $^{125}$I-glycoconjugate was prepared to form a radiolabeled protein in which radiolabeled tyramine is bound to carbohydrate which is bound covalently to the protein. This type of radioligand is stable despite exposure to lysosomal enzymes or acidic conditions. Furthermore, it does not alter functional properties of the labeled protein. In the labeling procedure, dilactitol tyramine (10 nmol in 24 $\mu$l, in 0.5 M potassium phosphate, pH 7.7) was initially radioiodinated by placing the label in an iodogen (Pierce Chemical Company, Rockford, Illinois) coated tube with Na$^{125}$I (1 mCi in 10 $\mu$l, Amersham). The reaction mixture was allowed to stand for 1 hour at room temperature with occasional vortexing. Contents of the tube were transferred to another tube containing galactose oxidase (4 units in 2 $\mu$l, Sigma) and incubated for 45 minutes at 37°C. Sodium cyanoborohydride (3 $\mu$l of a 1 M solution in potassium phosphate buffer) and 2 mg of bovine or human serum albumin (fraction V, Sigma) in 200 $\mu$l of physiological saline were added to the tube, followed by additional incubation for 45 minutes at 37°C. After incubation, 50 $\mu$l of 1 M ammonium bicarbonate was added to the tube. $^{125}$I-dilactitol tyramine-albumin was separated from $^{125}$I-dilactitol tyramine by Sephacryl S300 chromatography on a 0.9 x 30.0 cm column and dialysis against physiological saline. Coupled protein was concentrated with Aquacide and sterilized by filtration through a 0.45 um membrane filter. An aliquot was removed for determination of protein content, specific radioactivity, and precipitation by trichloroacetic acid.

Quantification of Absorption of Proteins after Intramuscular Injection with or without the Absroption Enhancing Agent: Radiolabeled BSA and HSA in plasma were quantified by assay of radioactivity in a well counter of aliquots of plasma obtained at selected intervals after injection and in precipitates formed by acidification of the plasma with four parts 10% trichloroacetic acid (TCA). Enhancement of absorption of BSA and HSA proteins after intramuscular injection was demonstrated in New Zealand rabbits. Protein solutions were injected manually through a 25 guage needle into skeletal muscle in the thigh, exposed while the animals were lightly anethetized.

Enhancement of Absorption: Radiolabeled albumin was administered to rabbits in 1 ml aliquots with 163.4 to 289.9 million counts per minute associated with microgram quantities of protein (specific radioactivity = 1.74 to 3.02 mCi/mg). Proteins were administered in either 0.9% NaCl solutions or an absorption enhancing solution of 0.9% NaCl supplemented with hydroxylamine as the hydrochloride in concentrations of 43.75 to 175 mg/ml.

Enhancement of absorption of protein was demonstrable with radiolabeled bovine serum albumin as well (24 determinations). Peak radioactivity in TCA precipitates from plasma averaged only 3204 counts per minutes per ml when the protein was injected intramuscularly in rabbits in saline alone. In contrast, TCA precipitable radioactivity averaged 16.580 counts per minute per ml of plasma with 80% of peak values attained 5 minutes after intramusuclar injection with absorption enhanced by hydroxylamine hydrochloride. Similarly, after injection of radiolabeled human serum albumin, TCA precipitable radioactivity averaged only 4176 counts per ml of plasma during the first 30 minutes after intramusuclar injection without enhancement of absorption. In contrast, TCA precipitable radioactivity averaged 32,868 counts per minute per ml of plasma during the first 30 minutes after intramuscular injection with absorption enhanced by inclusion of 43.8 mg of hydroxylamine as the hydrochloride per ml of injectate (12 determinations). Furthermore, 75% of peak radioactivity was evident 5 minutes after injection. The subsequent increment of plasma radioactivity after the first 5 minutes was attributable to continued absorption of radiolabeled albumin at a rate more rapid than its slow rate of clearance from the circulation.

## Claims

**Claims for the following Contracting States : BE, CH, DE, FR, GB, IT, LI, LU, NL, SE**

1. A package containing (1) a protein having therapeutic properties and (2) an agent capable of enhancing the absorption of the protein in the blood in an amount effective to enhance the absorption of the protein when injected intramuscularly, wherein the absorption enhancing agent is a $C_{1-4}$ alkylamine, a di $C_{1-3}$ alkylamine, phenylethylamine, epinephrine, tyramine, mono-, di- or tri-ethanolamine, propanolamine, methoxyamine, ethylenediamine, diethylenetriamine, or non-toxic salts thereof.

2. A package according to claim 1, wherein the absorption enhancing agent is (1) a $C_{1-4}$ alkylamine, (2) a di $C_{1-3}$ alkylamine, (3) a non-toxic salt of $C_{1-4}$ alkylamine or (4) a non-toxic salt of a di $C_{1-3}$ alkylamine.

3. A package according to claim 2, wherein the absorption enhancing agent is (a) methylamine, (b) dimethylamine, (c) a non-toxic salt of methylamine, or (d) a non-toxic salt of dimethylamine.

4. A package according to claim 3, wherein the absorption enhancing agent is methylamine or a non-toxic salt of dimethylamine.

5. A package according to claim 3, wherein the absorption enhancing agent is methylaminehydrochloride.

6. A package according to claims 1 to 5, wherein the protein is t-PA.

**Claims for the following Contracting State : AT**

1. A process for the production of a package for enhancing the absorption of a protein having therapeutic properties when injected intramuscularly, characterized in that the protein is combined within a package with an absorption enhancing agent selected from a $C_{1-4}$ alkylamine, a di $C_{1-3}$ alkylamine, phenylethylamine, epinephrine, tyramine, mono-, di- or tri-ethanolamine, propanolamine, methoxyamine, ethylenediamine, diethylenetriamine, or non-toxic salts thereof.

2. A process according to claim 1, wherein the absorption enhancing agent is (1) a $C_{1-4}$ alkylamine, (2) a di $C_{1-3}$ alkylamine, (3) a non-toxic salt of $C_{1-4}$ alkylamine or (4) a non-toxic salt of a di $C_{1-3}$ alkylamine.

3. A process according to claim 2, wherein the absorption enhancing agent is (a) methylamine, (b) dimethylamine, (c) a non-toxic salt of methylamine, or (d) a non-toxic salt of dimethylamine.

4. A process according to claim 3, wherein the absorption enhancing agent is methylamine or a non-toxic salt of dimethylamine.

5. A process according to claim 3, wherein the absorption enhancing agent is methylaminehydrochloride.

6. A process according to claims 1 to 5, wherein the protein is t-PA.

**Patentansprüche**
**Patentansprüche für folgende Vertragsstaaten : BE, CH, DE, FR, GB, IT, LI, LU, NL, SE**

1. Packung, die (1) ein Protein mit therapeutischen Eigenschaften und (2) ein die Absorption des Proteins in das Blut verbesserndes Mittel in einer Menge enthält, die wirksam ist, die Absorption des Proteins bei intramuskulärer Injektion zu verbessern, wobei das die Absorption verbessernde Mittel ein $C_{1-4}$-Alkylamin, ein $C_{1-3}$-Dialkylamin, Phenylethylamin, Epinephrin, Tyramin, Mono-, Di- oder Triethanolamin, Propanolamin, Methoxyamin, Ethylendiamin, Diethylentriamin, oder ein nicht-toxisches Salz davon ist.

2. Packung nach Anspruch 1, in der das die Absorption verbessernde Mittel (1) ein $C_{1-4}$-Alkylamin, (2) ein $C_{1-3}$-Dialkylamin, (3) ein nicht-toxisches Salz eines $C_{1-4}$-Alkylamins oder (4) ein nicht-toxisches Salz eines $C_{1-3}$-Dialkylamins ist.

3. Packung nach Anspruch 2, in der das die Absorption verbessernde Mittel (a) Methylamin, (b) Dimethylamin, (c) ein nicht-toxisches Salz von Methylamin, oder (d) ein nichttoxisches Salz von Dimethylamin ist.

4. Packung nach Anspruch 3, in der das die Absorption verbessernde Mittel Methylamin oder ein nicht-toxisches Salz von Dimethylamin ist.

5. Packung nach Anspruch 3, in der das die Absorption verbessernde Mittel Methylaminhydrochlorid ist.

6. Packung nach den Ansprüchen 1 bis 5, in der das Protein t-PA ist.

**Patentansprüche für folgenden Vertragsstaat : AT**

1. Verfahren zur Herstellung einer Packung zur Verbesserung der Absorption eines Proteins mit therapeutischen Eigenschaften bei intramuskulärer Injektion, dadurch gekennzeichnet, daß man das Protein in einer Packung mit einem die Absorption verbessernden Mittel kombiniert, welches aus einem $C_{1-14}$-Alkyla-

min,einem Di-$C_{1-3}$-Alkylamin, Phenylethylamin, Epinephrin, Tyramin, Mono-, Di- oder Triethanolamin, Propanolamin, Methoxyamin, Ethylendiamin, Diethylentriamin oder nicht-toxischen Salzen derselben ausgewählt ist.

2. Verfahren nach Anspruch 1, in dem das die Absorption verbessernde Mittel (1) ein $C_{1-14}$-Alkylamin, (2) ein Di-$C_{1-3}$-Alkylamin, (3) ein nicht-toxisches Salz von $C_{1-4}$-Alkylamin oder (4) ein nicht-toxisches Salz eines Di-$C_{1-3}$-Alkylamins ist.

3. Verfahren nach Anspruch 2, in dem das die Absorption verbessernde Mittel (a) Methylamin, (b) Dimethylamin, (c) ein nicht-toxisches Salz von Methylamin oder (d) ein nicht-toxisches Salz von Dimethylamin ist.

4. Verfahren nach Anspruch 3, in dem das die Absorption verbessernde Mittel Methylamin oder ein nicht-toxisches Salz von Dimethylamin ist.

5. Verfahren nach Anspruch 3, in dem das die Absorption verbessernde Mittel Methylaminhydrochlorid ist.

6. Verfahren nach den Ansprüchen 1 bis 5, in denen das Protein t-PA ist.

**Revendications**
**Revendications pour les Etats contractants suivants : BE, CH, DE, FR, GB, IT, LI, LU, NL, SE**

1. Forme de présentation pharmaceutique, contenant (1) une protéine ayant des propriétés thérapeutiques et (2) un agent capable d'amplifier l'absorption de la protéine dans le sang, cet agent étant présent en une quantité efficace pour amplifier l'absorption de la protéine en cas d'injection intramusculaire, cet agent amplifiant l'absorption étant une alkylamine en $C_1$ à $C_4$, une dialkylamine (groupe alkyle en $C_1$ à $C_3$), la phényléthylamine, l'épinéphrine, la tyramine, la monoéthanolamine, la diéthanolamine ou la triéthanolamine, la propanolamine, la méthoxyamine, l'éthylènediamine, la diéthylènetriamine, ou un de leurs sels non toxiques.

2. Forme de présentation pharmaceutique selon la revendication 1, dans laquelle l'agent amplifiant l'absorption est (1) une alkylamine en $C_1$ à $C_4$, (2) une dialkylamine en $C_1$ à $C_3$, (3) un sel non toxique de l'alkylamine en $C_1$ à $C_4$, ou (4) un sel non toxique d'une dialkylamine en $C_1$ à $C_3$.

3. Forme de présentation pharmaceutique selon la revendication 2, dans laquelle l'agent amplifiant l'absorption est (a) la méthylamine, (b) la diméthylamine, (c) un sel non toxique de la méthylamine, ou (d) un sel non toxique de la diméthylamine.

4. Forme de présentation pharmaceutique selon la revendication 3, dans laquelle l'agent amplifiant l'absorption est la méthylamine ou un sel non toxique de la diméthylamine.

5. Forme de présentation pharmaceutique selon la revendication 3, dans laquelle l'agent amplifiant l'absorption est le chlorhydrate de méthylamine.

6. Forme de présentation pharmaceutique selon les revendications 1 à 5, dans laquelle la protéine est t-PA.

**Revendication pour l'Etat contractant suivant : AT**

1. Procédé pour la production d'une forme de présentation pharmaceutique pour amplifier l'absorption d'une protéine ayant des propriétés thérapeutiques en cas d'injection intramusculaire, procédé caractérisé en ce que la protéine est combinée, au sein d'un emballage de présentation pharmaceutique, avec un agent amplifiant l'absorption et qui est choisi parmi une alkylamine en $C_1$ à $C_4$, une dialkylamine (groupes alkyles) en $C_1$ à $C_3$, la phényléthylamine, l'épinéphrine, la tyramine, la monoéthanolamine, la diéthanolamine ou la triéthanolamine, la propanolamine, la méthoxyamine, l'éthylènediamine, la diéthylènetriamine ou un de leurs sels non toxiques.

2. Procédé selon la revendication 1, dans lequel l'agent amplifiant l'absorption est (1) une alkylamine en $C_1$ à $C_4$ ; (2) une dialkylamine dont chaque groupe alkyle est en $C_1$ à $C_3$, (3) un sel non toxique de l'alkylamine en $C_1$ à $C_4$, ou (4) un sel non toxique d'une dialkylamine en $C_1$ à $C_3$.

3. Procédé selon la revendication 2, dans lequel l'agent amplifiant l'absorption est (a) la méthylamine, (b) la diméthylamine, (c) un sel non toxique de la méthylamine, ou (d) un sel non toxique de la diméthylamine.

4. Procédé selon la revendication 3, dans lequel l'agent amplifiant l'absorption est la méthylamine ou un sel non toxique de la diméthylamine.

**5.** Procédé selon la revendication 3, dans lequel l'agent amplifiant l'absorption est le chlorhydrate de méthylamine.

**6.** Procédé selon les revendications 1 à 5, dans lequel la protéine est t-PA.